Europäisches Patentamt

(19) European Patent Office

Office européen des brevets

(11) Publication number: **0 132 303**
**B1**

# (12) EUROPEAN PATENT SPECIFICATION

(45) Date of publication of patent specification: 22.04.87

(21) Application number: 84304218.5

(22) Date of filing: 22.06.84

(51) Int. Cl.⁴: **C 07 C 67/36,** C 07 C 69/14, C 07 C 69/22, B 01 J 37/20, B 01 J 27/04

(54) Catalytic carboxylic acid ester homologation.

(30) Priority: 23.06.83 US 507306
23.06.83 US 507307

(43) Date of publication of application:
30.01.85 Bulletin 85/05

(45) Publication of the grant of the patent:
22.04.87 Bulletin 87/17

(84) Designated Contracting States:
BE DE FR GB NL

(56) References cited:
EP-A-0 064 429
DE-A-1 545 261
DE-A-3 016 715
GB-A-2 079 748

(73) Proprietor: CHEVRON RESEARCH COMPANY
525 Market Street
San Francisco California 94120 (US)

(72) Inventor: Current, Steven P.
1207 Ridgeview Heights
Novato, CA 94947 (US)

(74) Representative: Kosmin, Gerald Emmanuel et al
HASELTINE, LAKE & CO. Hazlitt House 28
Southampton Buildings Chancery Lane
London, WC2A 1AT (GB)

Courier Press, Leamington Spa, England.

# 0 132 303

## Description

This invention relates to a process for the homologation of carboxylic acid esters and is concerned with a process for the conversion of carboxylic acid esters to homologous carboxylic acid esters by reaction of the ester with hydrogen and carbon monoxide in the presence of a heterogeneous sulphided catalyst.

An article by M. Hidai et al. in Bull. Chem. Soc. Japan, volume 55, pages 3951—52 (1982) describes the homologation of methyl esters, in particular the conversion of methyl acetate to ethyl acetate, with synthesis gas in the presence of a homogeneous ruthenium-cobalt catalyst and a methyl iodide promoter.

European Patent Application Publication No. 31606 describes the preparation of carboxylic acids and esters from carboxylic acid esters having one less carbon atom, carbon monoxide and hydrogen in the presence of a catalyst containing a ruthenium compound, a Group II metal iodide and/or bromide and a further Group VIII metal compound.

European Patent Application Publication No. 31784 describes the preparation of alkyl carboxylates from lower homologues by reaction with carbon monoxide and hydrogen using a ruthenium, cobalt and iodide catalyst system.

European Patent Application Publication No. 46128 describes the hydrocarbonylation and/or carbonylation of alkyl carboxylates in the presence of ruthenium, cobalt, vanadium and an iodide promoter.

According to the present invention, there is provided a process for the conversion of carboxylic acid esters to homologous carboxylic acid esters, which comprises reacting a carboxylic acid ester having from two to twenty carbon atoms with hydrogen and carbon monoxide at a temperature in the range from 150 to 350°C and a pressure in the range from 500 to 5,000 psig (36 to 353 kg/cm$^2$) in the presence of a heterogeneous sulphided catalyst comprising nickel or cobalt, optionally in admixture with a co-catalyst selected from the elements of Group VIB of the Periodic Table.

The present invention is based on the discovery that carboxylic acid esters can be converted to useful higher homologues having at least one more carbon atom than the starting ester in improved yield and selectivity by utilizing a heterogeneous sulphided catalyst system.

An advantage of the process of the present invention lies in the fact that the heterogeneous catalyst employed is easier to separate from the reaction products than the homogeneous catalysts of the prior art.

In addition, it has been found that process of the present invention does not require the use of any soluble catalyst promoters. This is particularly advantageous, since the absence of a halide promoter in the system obviates the need for expensive corrosion resistant equipment.

Oxygen-containing carbon compounds which can be obtained with high selectivity by the process of the invention are carboxylic acid esters of the secondary products which may be formed therefrom under the reaction conditions in a subsequent reaction, for example, transesterification, reduction, hydrolysis, condensation or dehydration.

Illustrative of a typical batch procedure, in the process of the invention the ester is charged to a high pressure reactor, and then there is introduced a heterogeneous sulphided catalyst system of Group VIB of the Periodic Table. The reactor is pressurized with a mixture containing carbon monoxide and hydrogen and heated for a suitable length of time to give the desired conversion. Liquid and gaseous products and reactants can be easily separated from the catalyst by filtration, distillation or other methods. Unreacted starting materials can be recycled. The products can be isolated by means of one of a number of known methods, including distillation. In some cases it may be advantageous to further process the products. For example, ethyl propionate can be easily hydrolyzed to propionic acid.

The process of the present invention can also be run in a continuous fashion. This is particularly advantageous as the catalyst is not soluble in the reaction medium. A number of reactor configurations are suitable including fixed or fluid beds, slurry beds or stirred tank reactors. As with a batch reaction, unreacted starting materials can be easily recycled and, if desired, the products can be further processed.

The carboxylic acid esters suitable for use in the present invention will generally contain from two to twenty, preferably two to six, carbon atoms. Preferred carboxylic acid esters include methyl acetate and ethyl acetate. If desired, the reactant ester may be diluted with an ester-miscible solvent such as dioxane, tetrahydrofuran or N-methylpyrolidinone. When methyl acetate is used as the starting ester, the reaction product predominantly formed is ethyl acetate, with lesser amounts of acetic acid, methanol and ethanol. When ethyl acetate is used as the starting ester, the reaction product predominantly formed is ethyl propionate.

The heterogeneous sulphided catalyst system employed in the present process comprises a composite of sulphides of a nickel or cobalt component and, optionally, a Group VIB component. Group VIB co-catalysts suitable for admixture with the nickel or cobalt component include chromium, molybdenum and tungsten. A particularly preferred catalyst system comprises nickel or cobalt and molybdenum. In addition, the catalyst system may optionally contain phosphorus or silicon.

In carrying out the reaction, it is usually desirable, although not essential, to place the catalyst on a support. Generally, the support should be a solid, inert material which is relatively insoluble in the solvent employed. Suitable supports include various treated or untreated organic and inorganic supports. Included among these are synthetic and naturally occurring polymers, alumina, silica, titania, silica-alumina, zeolites, glass and carbon. Particularly preferred supports are alumina and silica-alumina.

2

The metals may be added to the support using a method known in the art, such as by impregnation or co-precipitation. The method of loading the catalyst on the support will depend on the nature and composition of the support. Generally, the most convenient method of depositing the metals on the support is by adding a solution of metal salts to the support and subsequently converting them to an insoluble form.

An especially suitable catalyst precursor may be prepared by impregnating alumina with an aqueous or organic solution of the metal salts, either together or sequentially, followed by drying and calcining to give the metal oxides.

The catalyst may be converted to its active sulphide form by any of a number of conventional procedures. Treatment with hydrogen sulphide or other sulphur-containing compounds such as carbon disulphide, dimethyl disulphide or sulphur, in the presence of hydrogen or synthesis gas is effective. This treatment can be either prior to or concurrent with the ester carbonylation reaction.

In the process of the present invention carboxylic acid esters are reacted with carbon monoxide and hydrogen (synthesis gas). Synthesis gas produced by the reaction of carbonaceous material with water is suitable. Mixtures of, for example, carbon dioxide and hydrogen, or carbon monoxide and water, may also be employed. Whether introduced originally, or produced *in situ* under processing conditions, the reaction elements of carbon monoxide and hydrogen are required for the reaction.

The relative molar quantities of carbon monoxide and hydrogen present during the reaction can vary in the range from 10:1 to 1:10, and preferably in the range from 3:1 to 1:3. An inert diluent gas such as nitrogen or helium may be included if desired.

The carbonylation reaction requires a relatively high pressure for optimum selectivity and yield of product. The pressure should be maintained in the range from 500 to 5,000 psig (36 to 353 $kg/cm^2$), and preferably in the range from 800 to 2000 psig (57 to 142 $kg/cm^2$).

The reaction is conducted at a temperature in the range from 150 to 350°C, and preferably in the range from 190 to 290°C.

The time that the reactants are in contact with the catalyst will be dependent, among other factors, on the temperature, pressure, ester reactant, catalyst, reactor configuration and the desired level of conversion.

The solid catalyst can be easily separated from the generally liquid and gaseous reaction products and unreacted starting materials by, for example, filtration, centrifugation, settling out or distillation. The catalyst can be reused in a subsequent reaction. Unreacted starting materials can be separated from reaction products and are suitable for recycle in the process.

The products of the reaction, which can be isolated by one of a number of well-known methods, such as distillation, are generally useful as solvents or chemical intermediates. In some cases it may be advantageous to further process the reaction products by well-known means to form other useful products. For example, ethyl propionate can be hydrolyzed to propionic acid.

The following Examples are provided to illustrate the invention.

Example 1

An 18 ml stainless steel reactor was charged with 5.0 ml of methyl acetate and 0.5 g of a catalyst comprising nickel (6%) and molybdenum (15%) oxides, supported on silica-alumina, that had been pretreated with 10% hydrogen sulphide in hydrogen at 325°C. Also included was 0.10 ml of 1,4-dioxane to serve as an internal standard for gas chromatography analysis. The reactor was pressurized with 900 psi (63 $kg/cm^2$) of a 2:1 mixture of hydrogen and carbon monoxide and heated with shaking at 240°C for four hours. Analysis of the liquid produce indicated the formation of ethyl acetate (2.2 mmol) and acetic acid (4.4 mmol) as major products. Lesser amounts of ethanol and methyl formate were also formed.

Example 2

A 300 ml stainless steel autoclave was charged with 100 ml of ethyl acetate and 9.79 g of a catalyst comprising nickel (3%) and molybdenum (15%) oxides, supported on alumina, that had previously been treated with 10% hydrogen sulphide in hydrogen at 325°C. The reactor was heated to 250°C and charged with a 2:1 mixture of hydrogen and carbon monoxide to give a pressure of 1500 psi (105 $kg/cm^2$). After six hours of heating, the reactor was cooled. Analysis by gas chromatography indicated the following major reaction products:

| | |
|---|---|
| Ethanol | 115 mmol |
| Ethyl Propionate | 17 mmol |
| Acetic Acid | 32 mmol |
| N-Propyl Acetate | 8 mmol |
| Propionic Acid | 5 mmol |

Example 3

The conditions of Example 2 were repeated using 10.24 g of catalyst and a final reactor pressure of 2500 psi (176 $kg/cm^2$). Analysis by gas chromatography indicated the following major reaction products:

| Ethanol | 121 mmol |
|---|---|
| Ethyl Propionate | 52 mmol |
| Acetic Acid | 68 mmol |
| N-Propyl Acetate | 10 mmol |
| Propionic Acid | 11 mmol |

Example 4

A 300 ml autoclave was charged with 100 ml of methyl propionate and 9.94 g of a catalyst comprising nickel (3%) and molybdenum (15%) oxides, supported on alumina, that had been previously treatd with 10% hydrogen sulphide in hydrogen at 325°C for 2.75 hours. The autoclave was sealed, charged with a 2:1 mixture of hydrogen and carbon monoxide to give a final pressure of 2625 psi (185 kg/cm$^2$), and heated to 250°C. After six hours, the reactor was cooled and pressure released. Analysis of the liquid products indicated the following major products:

| Methyl Acetate | 61 mmol |
|---|---|
| Ethyl Acetate | 15 mmol |
| Ethanol | 13 mmol |
| Ethyl Propionate | 19 mmol |
| Methyl Butyrate | 6 mmol |
| Acetic Acid | 18 mmol |
| Propionic Acid | 133 mmol |

Examples 5—9

A stainless steel reactor tube was charged with 6.89 g of a catalyst comprising nickel (3.1%), molybdenum (12.9%) and phosphorus (2.4%) oxides supported on alumina. The catalyst was treated with 10% hydrogen sulphide in hydrogen at 325°C for three hours, then purged with nitrogen. Synthesis gas, comprising two parts hydrogen and one part carbon monoxide, and methyl acetate were passed over the catalyst at the rate, pressure, temperature and time indicated in Table 1. The methyl acetate contained a small amount of toluene to serve as a standard for gas chromatography analysis. The GHSV reported is for the exit gases. The average rates of product formation, as determined by on-line gas chromatography, are also reported in Table 1.

**0 132 303**

TABLE 1

| | Example 5 | Example 6 | Example 7 | Example 8 | Example 9 |
|---|---|---|---|---|---|
| **Reaction conditions** | | | | | |
| Temperature, °C | 239 | 248 | 249 | 250 | 251 |
| Pressure, kg/cm$^2$ | 107 | 107 | 141 | 141 | 141 |
| GHSV (off gas) | 2096 | 2101 | 2325 | 2283 | 849 |
| LHSV (feed) | .66 | .66 | .67 | .34 | .33 |
| Time at Condition, hr | 23.34 | 16.08 | 24.25 | 24.25 | 19.76 |
| **Products, MMOL/HR** | | | | | |
| Methane | 1.74 | 2.80 | 3.08 | 3.52 | 4.48 |
| Carbon Dioxide | .00 | 1.69 | 1.57 | 2.17 | 1.78 |
| Water | 8.74 | 10.30 | 11.86 | 12.50 | 10.85 |
| Dimethyl Ether | 4.44 | 5.54 | 5.08 | 4.26 | 2.99 |
| Methanol | 4.92 | 12.30 | 11.83 | 7.04 | 6.69 |
| Methyl Ethyl Ether | 1.05 | 1.79 | 1.65 | 2.42 | 1.45 |
| Ethanol | 4.93 | 8.05 | 7.99 | 8.82 | 7.83 |
| Methyl Acetate (feed) | 35.38 | 27.43 | 27.85 | 7.39 | 9.26 |
| Ethyl Acetate | 9.15 | 9.82 | 8.66 | 4.70 | 5.37 |
| Acetic Acid | .00 | .57 | .70 | .71 | .77 |
| Toluene, internal std. | 2.19 | 2.19 | 2.19 | 1.10 | 1.10 |

Example 10

An 18 ml stainless steel reactor was charged with 5.0 ml of methyl acetate and 0.5 g of a catalyst comprising cobalt (3%) and molybdenum (12%) oxides, supported on alumina, that had been pretreated with 10% hydrogen sulphide in hydrogen at 325°C. Also included was 0.10 ml of 1,4-dioxane to serve as a gas chromatography reference. The reactor was pressurized to 900 psi (63 kg/cm$^2$) with a 2:1 mixture of hydrogen and carbon monoxide and heated with shaking to 240°C for four hours. Analysis of the liquid product indicated the formation of ethyl acetate (2.3 mmol) and acetic acid (3.1 mmol) as major products. Lesser amounts of ethanol and methyl formate were also formed.

Examples 11—15

A stainless steel reactor tube was charged with 7.23 g of a catalyst comprising cobalt (3.2%), molybdenum (12.4%) and phosphorus (1.7%) oxides supported on alumina. The catalyst was treated with 10% hydrogen sulphide in hydrogen at 325°C until water formation ceased, then purged with nitrogen. Synthesis gas, comprising two parts hydrogen and one part carbon monoxide, and methyl acetate were passed over the catalyst at the rate, pressure, temperature and time indicated in Table 2. The methyl acetate contained a small amount of toluene to serve as a standard for gas chromatography analysis. The GHSV reported is for the exit gases. The average rates of product formation, as determined by on-line gas chromatography, are also reported in Table 2.

5

TABLE 2

| | Example 11 | Example 12 | Example 13 | Example 14 | Example 15 |
|---|---|---|---|---|---|
| Reaction conditions Temperature, °C | 240 | 250 | 250 | 250 | 250 |
| Pressure, kg/cm² | 110.7 | 111 | 144.8 | 141 | 143 |
| GHSV (off gas) | 2375 | 2302 | 2555 | 1935 | 1238 |
| LHSV (feed) | .71 | .65 | .71 | .36 | .41 |
| Time at Condition, hr | 17.92 | 23.83 | 21.25 | 23.92 | 26.22 |
| Products, MMOL/HR Methane | 1.15 | 2.08 | 2.63 | 4.04 | 5.00 |
| Carbon Dioxide | .78 | 1.74 | 1.76 | 2.27 | 2.19 |
| Water | 10.65 | 14.91 | 15.27 | 12.56 | 12.59 |
| Dimethyl Ether | 2.06 | 3.09 | 3.72 | 2.91 | 2.39 |
| Methanol | 5.52 | 7.50 | 8.28 | 5.15 | 5.23 |
| Methyl Ethyl Ether | .33 | .80 | 1.08 | 1.51 | 1.20 |
| Ethanol | 2.07 | 3.74 | 4.48 | 5.99 | 6.11 |
| Methyl Acetate (feed) | 41.06 | 33.84 | 31.19 | 9.12 | 9.46 |
| Ethyl Acetate | 8.41 | 10.16 | 10.41 | 5.54 | 5.68 |
| Acetate Acid | 1.55 | 2.00 | 1.56 | 1.57 | 2.29 |
| Toluene, internal std. | 2.19 | 2.19 | 2.19 | 1.10 | 1.10 |

## Claims

1. A process for converting a carboxylic acid ester to a homologous carboxylic acid ester, which comprises reacting a carboxylic acid ester having from two to twenty carbon atoms with hydrogen and carbon monoxide at a temperature in the range from 150 to 350°C and a pressure in the range from 500 to 5,000 psig (36 to 353 kg/cm²) in the presence of a heterogeneous sulphided catalyst comprising nickel or cobalt, optionally in admixture wiht a co-catalyst selected from the elements of Group VIB of the Periodic Table.

2. A process according to Claim 1, wherein the co-catalyst is molybdenum.

3. A process according to Claim 1 or 2, wherein the sulphided catalyst further comprises phosphorus or silicon.

4. A process according to Claim 1, 2 or 3, wherein the sulphided catalyst is present on a support.

5. A process according to Claim 4, wherein the support is alumina or silica-alumina.

6. A process according to any preceding claim, wherein methyl acetate is converted to ethyl acetate.

7. A process according to any one of Claims 1 to 5, wherein ethyl acetate is converted to ethyl propionate.

## Patentansprüche

1. Verfahren zur Umwandlung eines Carbonsäureesters in einen homologen Carbonsäureester, dadurch gekennzeichnet, daß ein Carbonsäureester mit 2 bis 20 Kohlenstoffatomen mit Wasserstoff und Kohlenmonoxid bei einer Temperatur im Bereich von 150 bis 350°C und einem Druck im Bereich von 500 bis 5000 psig (36 bis 353 kg/cm²) in Gegenwart eines heterogenen sulfidierten Katalysators aus Nickel oder Kobalt, gegebenenfalls in Mischung mit einem Co-katalysator, ausgewählt aus den Elementen der Gruppe VIB des Periodischen Systems der Elemente, umgesetzt wird.

2. Verfahren nach Anspruch 1, dadurch gekennzeichnet, daß der Cokatalysator aus Molybdän besteht.

3. Verfahren nach Anspruch 1 oder 2, dadurch gekennzeichnet, daß der sulfidierte Katalysator außerdem Phosphor oder Silizium enthält.

4. Verfahren nach Anspruch 1, 2 oder 3, dadurch gekennzeichnet, daß der sulfidierte Katalysator auf einem Träger vorliegt.

5. Verfahren nach Anspruch 4, dadurch gekennzeichnet, daß der Träger aus Aluminiumoxid oder Siliziumdioxid/Aluminiumoxid besteht.

6. Verfahren nach einem der vorhergehenden Ansprüche, dadurch gekennzeichnet, daß Methylacetat in Ethylacetat umgewandelt wird.

7. Verfahren nach einem der Ansprüche 1 bis 5, dadurch gekennzeichnet, daß Ethylacetat in Ethylpropionat umgewandelt wird.

## Revendications

1. Procédé pour convertir un ester d'acide carboxylique en un ester d'acide carboxylique homologue, qui consiste à faire réagir un ester d'acide carboxylique ayant 2 à 20 atomes de carbone avec l'hydrogène et l'oxyde de carbone à une température comprise dans l'intervalle de 150 à 350°C et à une pression manométrique comprise dans l'intervalle de 500 à 5000 lb/in$^2$ (36 à 353 kg/cm$^2$) en présence d'un catalyseur sulfuré hétérogène comprenant du nickel ou du cobalt, éventuellement en mélange avec un cocatalyseur choisi parmi les éléments du Groupe VIB du Tableau Périodique.

2. Procédé suivant la revendication 1, dans lequel le cocatalyseur est le molybdène.

3. Procédé suivant la revendication 1 ou 2, dans lequel le catalyseur sulfuré comprend en outre du phosphore ou du silicium.

4. Procédé suivant la revendication 1, 2 ou 3, dans lequel le catalyseur sulfuré est présent sur un support.

5. Procédé suivant la revendication 4, dans lequel le support est de l'alumine ou un mélange silicealumine.

6. Procédé suivant l'une quelconque des revendications précédentes, dans lequel l'acétate de méthyle est converti en acétate d'éthyle.

7. Procédé suivant l'une quelconque des revendications 1 à 5, dans lequel l'acétate d'éthyle est converti en propionate d'éthyle.